# EUROPEAN PATENT SPECIFICATION

(11) **EP 1 741 381 B1**
(45) Date of publication and mention of the grant of the patent: **10.09.2008**
(21) Application number: 06013929.2
(22) Date of filing: 05.07.2006
(51) Int. Cl.: A61B 1/00

(54) **Balloon control device for endoscopic apparatus**
Steuerungsvorrichtung für einen Ballon für Endoskop
Appareil de commande d'un ballon pour endoscope

(30) Priority: 06.07.2005 JP 2005197681
(43) Date of publication of application: 10.01.2007
(73) Proprietor: Fujinon Corporation, Saitama-shi, Saitama (JP)
(72) Inventor: Sekiguchi, Tadashi, Kita-ku, Saitama-shi, Saitama (JP); Kumekawa, Takashi, Kita-ku, Saitama-shi, Saitama (JP)
(74) Representative: Klunker . Schmitt-Nilson . Hirsch

(56) References cited:
- EP-A- 0 487 175
- EP-A- 1 547 640
- PATENT ABSTRACTS OF JAPAN vol. 2003, no. 02, 5 February 2003 (2003-02-05) & JP 2002 301019 A (YAMAMOTO HIRONORI), 15 October 2002 (2002-10-15)

## Description

### BACKGROUND OF THE INVENTION

### Field of the Invention

The present invention relates to a balloon control device for an endoscopic apparatus, in particular, a balloon control device which controls balloons in an endoscopic apparatus for observing lower gastrointestinal tract such as small intestine or colon.

### Description of the Related Art

In inserting an inserting section of an endoscope into lower gastrointestinal tract such as small intestine, if the inserting section is simply pushed down there, the pushing power cannot be transmitted enough to a distal end of the inserting section and it is difficult to insert the inserting section into the lower part. For example, when an inserting section is unnecessarily bent or deflected, the inserting section cannot be inserted further into the lower part. So, methods have been proposed for preventing any excess bend or deflection of an inserting section by inserting the inserting section of an endoscope into a body cavity with an insertion assisting tool being covering the inserting section for guiding the inserting section in the body cavity.

For example, Japanese Patent Application Laid-Open No. 2002-301019, as well as EP 1 547 640 A1, describes an endoscopic apparatus which comprises an endoscope having an inserting section with a first balloon at the distal end portion thereof and an insertion assisting tool (also called as an over tube or sliding tube) with a second balloon at the distal end portion thereof. Expanding the first balloon and the second balloon allows the inserting section and the insertion assisting tool to be fixed at points in intestinal tract such as small intestine. Thus, the first balloon and the second balloon are repeatedly expanded and contracted to alternately insert the inserting section and the insertion assisting tool, so that the inserting section can be inserted to the lower intestinal tract such as small intestine which is complicatedly bent.

The first balloon and the second balloon described above are controlled to be expanded and contracted by a balloon control device. The balloon control device has a device body to which a tube is coupled, and the tube has a distal end which is in communication with the first balloon and the second balloon. To the coupling between the device body and the tube is provided with a trap which prevents liquids such as body fluid in the tube from flowing into the device body. For the trap, a bottle device has been conventionally used, and in recent years, a device using a filter for gas and liquid separation has been proposed.

EP 0 487 175 A1 discloses a laparoscopic trocar with a selectively lockable sleeve, which can be fixed to an abdominal wall with the aid of a balloon. A syringe port for inflation of a balloon is mounted to sleeve handle with a distensible coupling bulb.

### SUMMARY OF THE INVENTION

With use of a filter for the trap, however, there is a problem that a resistance in a tube path is increased, and that this causes a large difference between the pressure in the tube path in the device body and the pressure in a balloon. A balloon control device typically includes a device body with a pressure sensor provided therein which measures a pressure in a tube path in the device body, and controls the expansion and contraction of the balloons by supplying and sucking air based on the measured value. If there is a large difference between the pressure in the tube path in the device body and the pressure in a balloon, the measured value by the pressure sensor is different from an actual inner pressure of a balloon, which results in an inaccurate control of the inner pressure of the balloon. That is, a supply or suction of fluid to and from a balloon by the device body rapidly changes a pressure in the tube path of the device body, and the value measured by the pressure sensor quickly reaches a preset value before the inner pressure of the balloon reaches the preset value, thereby the inner pressure of the balloon cannot be accurately controlled.

The present invention is made in view of the above problems, and one object of the present invention is to provide a balloon control device for an endoscopic apparatus which accurately controls an inner pressure of a balloon by reducing a difference between a pressure in the balloon and a pressure in a tube path.

To achieve the above object, a first aspect of the present invention provides a balloon control device for an endscopic apparatus, which supplies and sucks a fluid to and from either a balloon mounted to an inserting section of the endscopic apparatus or a balloon mounted to an insertion assisting tool covering the inserting section for assisting the insertion of the inserting section, comprising a buffering section in a tube path through which a fluid flows so as to buffer a change in pressure of the fluid.

According to the first aspect of the present invention, because the buffering section buffers a change in pressure of the fluid, a rapid change in pressure in a tube path can be restrained. This reduces a difference between a pressure in a tube path and an inner pressure of a balloon, thereby the inner pressure of a balloon can be accurately controlled by controlling the pressure of the tube path by the balloon control device.

The first aspect of the present invention further provides the balloon control device, wherein a pressure sensor is connected to the tube path through which the fluid flows via a manifold, and the buffering section is provided to the manifold. So, according to the first aspect of the present invention, as the buffering section is provided to a manifold, the device can be compact compared to the one having a buffering section as a separate member. Also, as the buffering section is provided to a manifold which connects a pressure sensor to a tube path, a measured value by the pressure sensor is accurate with a smaller margin of error to an actual inner pressure of a balloon. Thus, the balloon control device can accurately control an inner pressure of a balloon.

A second aspect of the present invention provides a balloon control device according to the first aspect, wherein a filter for gas and liquid separation is disposed in the tube path, and the buffering section is disposed across the filter on a side opposite to the balloon. The present invention is especially effective to a balloon control device including such a filter for gas and liquid separation. Because a filter for gas and liquid separation increases a resistance in a tube path across the filter for gas and liquid separation, a difference between a pressure in a tube path downstream of the filter toward a balloon and a pressure in a tube path upstream of the filter is increased. An application of the present invention to such a device causes the difference between the pressures to be reduced, which allows an inner pressure of a balloon to be accurately controlled.

A third aspect of the present invention provides a balloon control device according to any one of the first aspect to the second aspect, wherein the buffering section is provided within the device body.

According to the present invention, as a buffering section is provided in a tube path through which a fluid flows and the buffering section buffers a change in pressure of the fluid and restrains a rapid change in a pressure of the tube path, a difference between a pressure of the tube path and a pressure in a balloon can be reduced, which allows an inner pressure of the balloon to be accurately controlled.

### BRIEF DESCRIPTION OF THE DRAWINGS

Fig. 1 is a system configuration diagram of an endoscopic apparatus according to the present invention;
Fig. 2 is a perspective diagram to show a distal end of an inserting section of an endoscope;
Figs. 3A to 3J are diagrams to illustrate a method of operating an endoscopic apparatus according to the present invention;
Fig. 4 is a block diagram to show an inner structure of a balloon control device;
Figs. 5A and 5B are diagrams to show an air storing section;
Fig. 6 is a flow chart to schematically show operations of a sequencer or control circuit of Fig. 4;
Fig. 7 is a flow chart to illustrate operations of the depressurizing process of Fig. 6;
Fig. 8 is a flow chart to illustrate operations of the pressurizing process of Fig. 6;
Fig. 9 is a flow chart to illustrate operations of the pausing process of Fig. 6; and
Figs. 10A and 10B are diagrams to show a manifold having an air storing section.

### DETAILED DESCRIPTION OF THE PREFERRED EMBODIMENTS

Now, a preferred embodiment of a balloon control device for an endoscopic apparatus according to the present invention will be described in detail with reference to the accompanying drawings.

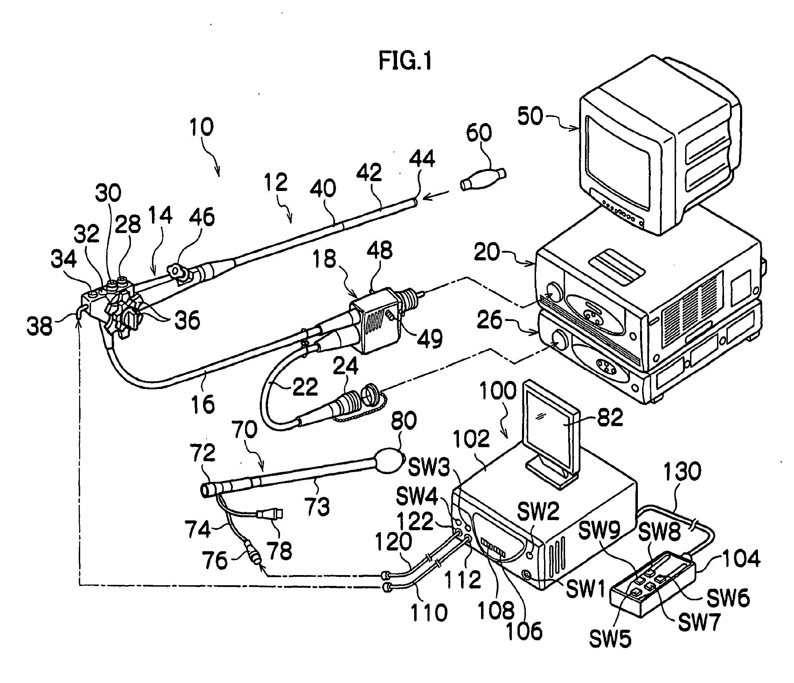

Fig. 1 is a system configuration diagram to show an embodiment of an endoscopic apparatus to which a balloon control device is applied according to the present invention. As shown in Fig. 1, an endoscopic apparatus generally comprises an endoscope 10, an insertion assisting tool 70, and a balloon control device 100.

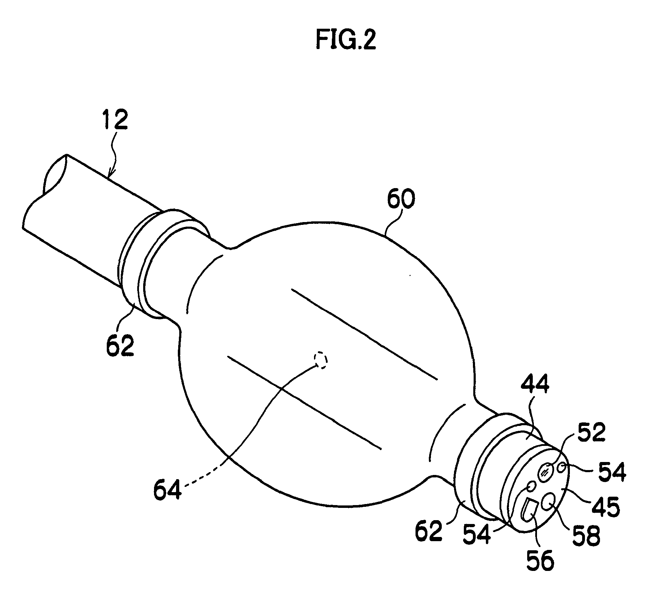

The endoscope 10 comprises a hand-held control section 14 and an inserting section 12 connected to the hand-held control section 14 to be inserted into a body cavity. The hand-held control section 14 is connected to a universal cable 16 having an end which is provided with an LG connector 18. The LG connector 18 is removably coupled to a light source device 20 which sends an illumination light to an illumination optical system 54 which will be explained below (see Fig. 2). The LG connector 18 is connected to an electric connector 24 via a cable 22, the electric connector 24 being removably coupled to a processor 26.

To the hand-held control section 14, an air and water supply button 28, a suction button 30, a shutter button 32, and a function switch button 34 are arranged in a line, and a pair of angle adjustment knobs 36 are also provided therewith. The hand-held control section 14 has a rear end in which an air inlet for balloon 38 is formed with a pipe which is bent into an L shape. A supply or suction of air through the air inlet for balloon 38 makes a first balloon 60 expanded or contracted, which will be explained below.

The inserting section 12 comprises a soft portion 40, a curved portion 42, and a distal end portion 44, starting from the hand-held control section 14, and the curved portion 42 is remotely controlled by turning the angle adjustment knobs 36 at the hand-held control section 14. This control allows the distal end portion 44 to be directed to a desired direction.

As shown in Fig. 2, the distal end portion 44 has a front surface 45 where an observation optical system 52, illumination optical systems 54, 54, an air and water supply nozzle 56, and a forceps port 58 are provided. A CCD (not shown) is mounted to the back of the observation optical system 52, and the CCD is supported by a substrate to which a signal cable (not shown) is connected. The signal cable is inserted therethrough into the inserting section 12, the hand held control section 14, the universal cable 16 and the like of Fig. 1 to be extended to the electric connector 24 which is connected to the processor 26. Thus, an observation image which is obtained by the observation optical system 52 is focused on a light sensitive surface of the CCD to be converted into an electrical signal, which is output through a signal cable to the processor 26 where the electrical signal is converted into an image signal. In this way, a picture image from observation is displayed on a monitor 50 which is in connection with the processor 26.

Behind the illumination optical systems 54 of Fig. 2, light guides (not shown) are disposed with the outputting ends thereof facing to the illumination optical systems 54. The light guides are inserted therethrough into the inserting section 12, the hand held control section 14, the universal cable 16 and the like of Fig. 1 to dispose the inputting ends thereof in the LG connector 18. Thus, when the LG connector 18 is coupled to the light source device 20, an illumination light irradiated from the light source device 20 is transmitted through the light guides to the illumination optical systems 54 to be irradiated forward from the illumination optical systems 54.

The air and water supply nozzle 56 of Fig. 2 is in communication with a valve (not shown) which is controlled by the air and water supply button 28 of Fig. 1, and the valve is in turn in communication with an air and water supply connector 48 which is provided in the LG connector 18. The air and water supply connector 48 is connected with an air and water supply device (not shown) to supply air or water. Thus, an actuation of the air and water supply button 28 causes air or water to be ejected from the air and water supply nozzle 56 toward the observation optical system 52.

The forceps port 58 of Fig. 2 is in communication with a forceps inserting section 46. When a procedure tool such as a forceps is inserted from the forceps inserting section 46, the procedure tool can be pulled out from the forceps port 58. The forceps port 58 is in communication with a valve (not shown) which is controlled by the suction button 30, and the valve is in turn in communication with a suction connector 49 of the LG connector 18. Thus, with a suction device (not shown) connected to suction connector 49, an operation of the valve by the suction button 30 causes body fluid and the like to be sucked through the forceps port 58.

The inserting section 12 has an outer circumferential surface, and to the outer circumferential surface, a first balloon 60 is attached which is made of a resilient material such as rubber. The first balloon 60 is formed into a generally tubular shape having two deflated ends, and after the inserting section 12 is inserted therethrough into the first balloon 60 and the first balloon 60 is disposed at a desired position around the inserting section 12, as shown in Fig. 2, fixing rubber rings 62 are fit onto both the ends of the first balloon 60 so that the first balloon 60 is fixed around the inserting section 12.

An air vent 64 is formed in the outer circumferential surface of the inserting section 12 where the first balloon 60 is attached. The air vent 64 is in communication with the air inlet for balloon 38 which is provided in the hand held control section 14 of Fig. 1. The air inlet for balloon 38 is connected to the balloon controlling device 100 via a tube 110. Thus, supplying or sucking air by the balloon controlling device 100 allows the first balloon 60 to be expanded or contracted. The first balloon 60 is expanded into a generally spherical shape by air supply, and is contracted to stick around the outer circumferential surface of the inserting section 12 by air suction.

Meanwhile, the insertion assisting tool 70 shown in Fig. 1 comprises a tubular and rigid holding section 72 which is provided at the rear end of the insertion assisting tool 70 and a tube body 73 which is attached to the distal end of the holding section 72, and the inserting section 12 of the endoscope 10 described above is inserted from the holding section 72 into the tube body 73.

The tube body 73 comprises a flexible resin tube substrate which is formed of urethane for example, and the substrate has an outer circumferential surface and an inner circumferential surface which are coated by a hydrophilic coating material (a lubricant coating material). The hydrophilic coating material may be, for example, polyvinyl pyrrolidone, acrylic resin and silicon resin.

A second balloon 80 is attached closely to the distal end of the tube body 73. The second balloon 80 is formed into a tubular shape having two deflated ends, and is attached to the tube body 73 with the insertion assisting tool 70 being inserted therethrough, and is fixed there by winding a thread (not shown) around the ends. The second balloon 80 is in communication with a tube 74 which is adhered to the outer circumferential surface of the insertion assisting tool 70, and the tube 74 has a rear end to which a connector 76 is provided. The connector 76 is connected to a tube 120, and to the balloon controlling device 100 via the tube 120. Thus, supplying or sucking air by the balloon controlling device 100 allows the second balloon 80 to be expanded or contracted. The second balloon 80 is expanded into a generally spherical shape by air supply, and is contracted to stick around the outer circumferential surface of the insertion assisting tool 70 by air suction.

The insertion assisting tool 70 has a rear end in which an inlet 78 is formed. The inlet 78 is in communication with an opening (not shown) which is formed in the inner circumferential surface of the insertion assisting tool 70. Thus, a lubricant (e.g. water) can be supplied into the insertion assisting tool 70 by injecting the lubricant with a syringe or the like from the inlet 78. This reduces the friction between the inner circumferential surface of the insertion assisting tool 70 and the outer circumferential surface of the inserting section 12 in inserting the inserting section 12 into the insertion assisting tool 70, and enables a smooth relative movement between the inserting section 12 and the insertion assisting tool 70.

The balloon controlling device 100 supplies and sucks fluids such as air to and from the first balloon 60, and also supplies and sucks fluids such as air to and from the second balloon 80. The balloon controlling device 100 generally comprises a device body 102 and a hand held switch 104 for remotely controlling.

The device body 102 has a front side where a power switch SW1, a stop switch SW2, a first pressure indicator 106, a second pressure indicator 108, a first function stop switch SW3, and a second function stop switch SW4 are provided. Each of the first pressure indicator 106 and the second pressure indicator 108 is a panel to indicate a pressure value of the first balloon 60 and the second balloon 80 respectively, and the pressure indicators 106 and 108 indicate an error code in the event of failure such as a balloon burst.

The first function stop switch SW3 and the second function stop switch SW4 turn on and off a control system for endoscope A and a control system for insertion assisting tool B which will be described below, respectively, and when only one of the first balloon 60 and the second balloon 80 is used, one of the function stop switches SW3 and SW4 which corresponds to the unused balloon is operated to be turned off. In the turned off control system A or B, any supply and suction of air is completely stopped, and the pressure indicator 106 or 108 for the system is also turned off. The initial conditions of the systems and the like may be set by turning off both of the function stop switches SW3 and SW4. For example, a calibration for an atmosphere pressure is performed by holding down all of the switches SW5 to SW9 on the hand held switch 104 while both of the function stop switches SW3 and SW4 are turned off.

To the front of the device body 102 are connected an air supply and suction tube 110 and an air supply and suction tube 120, for the first balloon 60 and the second balloon 80 respectively. Backflow prevention units 112 and 122 are provided at the points where each of the tubes 110 and 120 is connected to the device body 102, which prevent any backflow of body fluid when the first balloon 60 or the second balloon 80 is burst. The backflow prevention units 112 and 122 are respectively structured by fitting a filter for gas and liquid separation into the inside of a hollow disk-like case (not shown) which is removably attached to the device body 102, so that the filter prevents any liquid flowing into the device body 102.

The pressure indicators 106 and 108, the function stop switches SW3 and SW4, and the backflow prevention units 112 and 122 are fixedly arranged for the endoscope 10 and for the insertion assisting tool 70. That is, the pressure indicator 106, the function stop switch SW3, and the backflow prevention unit 112 are arranged for the endoscope 10 on the right side relative to the pressure indicator 108, the function stop switch SW4, and the backflow prevention unit 122 for the insertion assisting tool 70, respectively.

Meanwhile, to the hand held switch 104, a stop switch SW5 which is similar to the stop switch SW2 on the device body 102, an ON/OFF switch SW6 to give a command to pressurize/depressurize the first balloon 60, a hold switch SW7 to maintain a pressure of the first balloon 60, an ON/OFF switch SW8 to give a command to pressurize/depressurize the second balloon 80, and a hold switch SW9 to maintain a pressure of the second balloon 80 are provided, and the hand held switch 104 is electrically connected to the device body 102 via a code 130. Also, a display section to display a condition of air supply or exhaust of the first balloon 60 and second balloon 80, which are not shown in Fig. 1, is provided to the hand held switch 104.

The balloon controlling device 100 configured as described above expands each balloon 60, 80 by supplying air, and maintains the expanded balloons 60, 80 by controlling the air pressure in the balloons at a constant value. The balloon controlling device 100 also contracts each balloon 60, 80 by sucking air, and maintains the contracted balloons 60, 80 by controlling the air pressure in the balloons at a constant value.

The balloon controlling device 100 is connected to a balloon exclusive monitor 82 which displays a pressure value and an expanded or contracted condition of each balloon 60, 80. A pressure value and an expanded or contracted condition of each balloon 60, 80 may be displayed on the monitor 50 by superimposing on an observation image obtained by the endoscope 10.

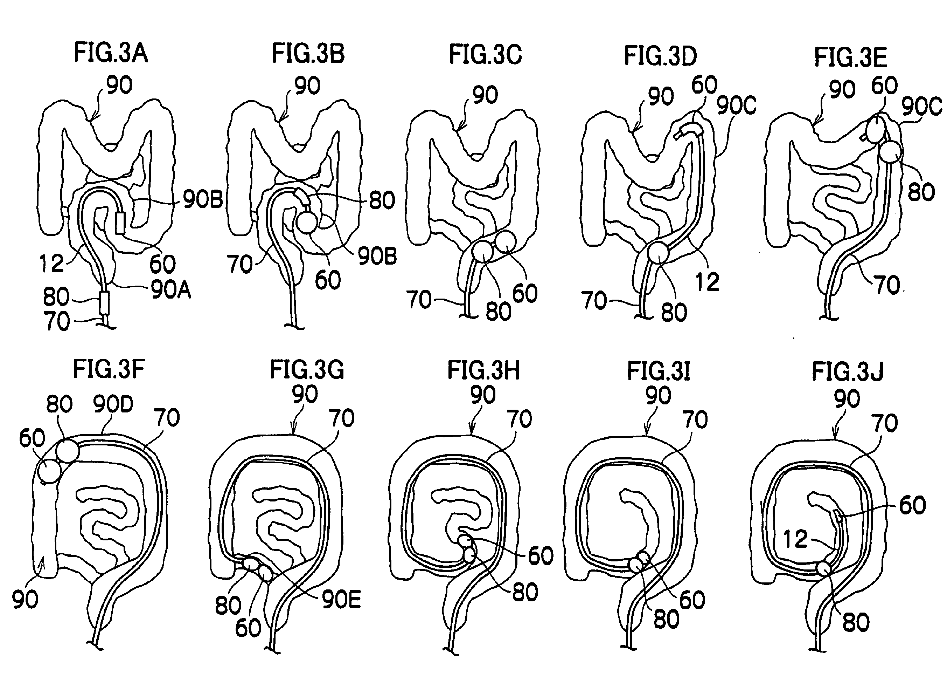

Now, a method of operating the endoscopic apparatus which is configured as described above will be explained with reference to Figs. 3A to 3J. Figs. 3A to 3J show an example of inserting an endoscopic apparatus by anal route, but the endoscopic apparatus may be inserted by oral route.

First, as shown in Fig. 3A, the inserting section 12 is inserted into intestinal tract (large intestine) 90 from anus 90A, with the inserting section 12 being inserted into the insertion assisting tool 70 (an inserting operation). In this operation, the first balloon 60. and the second balloon 80 are contracted.

Next, as shown in Fig. 3A, when the distal end of the inserting section 12 reaches sigmoid colon 90B, the first balloon 60 is expanded to fix the distal end of the inserting section 12 to the intestinal tract 90 (a fixing operation).

Then the insertion assisting tool 70 is pushed down to be inserted along the inserting section 12 (a pushing operation). As shown in Fig. 3B, when the distal end of the insertion assisting tool 70 comes close to the first balloon 60, the second balloon 80 is expanded by supplying air. This fixes the second balloon to the intestinal tract 90, which holds the intestinal tract 90 around the insertion assisting tool 70 via the second balloon 80 (a holding operation).

As shown in Fig. 3C, the insertion assisting tool 70 is drawn back to remove any excess deflection or bend of the intestinal tract 90 (a drawing back operation).

Next, the air in the first balloon 60 is sucked to contract the first balloon 60. As shown in Fig. 3D, the inserting section 12 is inserted into the upper part of the intestinal tract 90 (for example, the bending part of the upper end of descending colon 90C) (an inserting operation). Then, as described above, after the fixing operation by expanding the first balloon 60 and the pushing operation by pushing the insertion assisting tool 70 along the inserting section 12 are performed, the holding operation by expanding the second balloon 80 and the drawing operation by drawing the insertion assisting tool 70 are performed. This removes any excess deflection or bend of the intestinal tract 90, as shown in Fig. 3E.

This series of operations described above (an inserting operation, a fixing operation, a pushing operation, a holding operation, and a drawing back operation) is repeatedly performed, so that the distal end of the inserting section 12 can be inserted little by little further into the upper part of intestinal tract 90. And this insertion of the insertion assisting tool 70 can remove any excess deflection of the intestinal tract 90.

For example, Fig. 3F shows a state in which any excess deflection of the intestinal tract 90 is removed by inserting the distal end of the inserting section 12 to the end of transverse colon 90D and performing the drawing operation of the insertion assisting tool 70. Fig. 3G shows a state in which any excess deflection of the intestinal tract 90 is removed by further inserting the distal end of the inserting section 12 to ileum of small intestine 90E. Fig. 3H shows a state in which any excess deflection of the intestinal tract 90 is removed by further inserting the distal end of the inserting section 12 to the upper part of small intestine.

When these operations are repeated so as to insert the distal end of the inserting section 12 to the upper part of small intestine, the insertion assisting tool 70 curves in a loop as shown in Fig. 3I. So, pushing the inserting section 12 as shown in Fig. 3J allows the inserting section 12 to be further inserted to the upper part of intestinal tract 90.

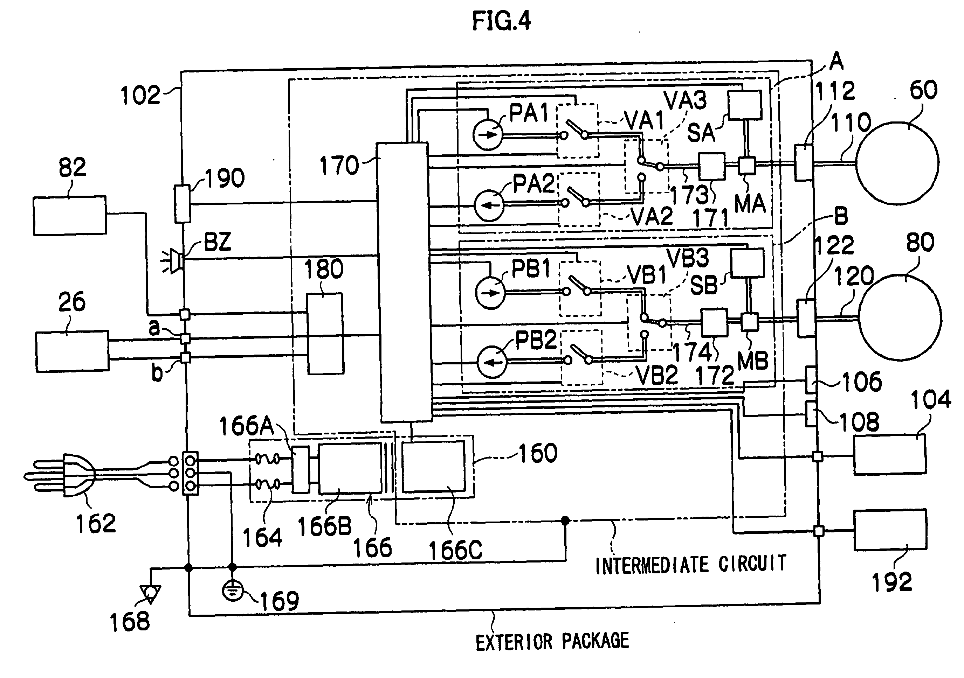

Now, an inner structure of the balloon control device 100 will be explained. Fig. 4 is a block diagram to show an embodiment of an inner structure of the balloon control device 100. As shown in Fig. 4, the balloon control device 100 comprises a device body 102 which generally includes a power circuit 160, a sequencer or control circuit 170, a control system for endoscope A, and a control system for insertion assisting tool B.

The power circuit 160 converts an incoming commercial power supply from a power plug 162 into a DC power supply of a desired voltage to supply it to each part of the device body 102, and includes a fuse 164 and a switching power supply 166. The switching power supply 166 includes a power switch 166A, a primary power source 166B, and a secondary power source 166C, and the primary power source 166B and the secondary power source 166C are separated by reinforced insulation from each other. Reference numeral 168 of Fig. 4 designates a potential equalization terminal, and reference numeral 169 designates a protective ground terminal, and the intermediate circuit shown by a dashed line is protectively grounded, and the exterior package shown by a solid line is protectively grounded.

A sequencer or control circuit 170 separately controls the control system for endoscope A and the control system for insertion assisting tool B based on various commands from the hand held switch 104, and detects a disorder such as an abnormal pressure and indicates the disorder by ringing a buzzer BZ for example upon the detection of the disorder.

The sequencer or control circuit 170 is connected to an image processing circuit 180, where the signals of measured values obtained by the pressure sensors SA and SB are converted into image signals. The processed signals are sent to a balloon exclusive monitor 82 which graphically displays an expanded or contracted condition of each balloon 60, 80. The image processing circuit 180 is connected to a processor 26, and when an observation image signal obtained by the endoscope 10 is input from an input terminal a, the image processing circuit 180 generates a signal on which an expanded or contracted condition of each balloon 60, 80 is superimposed and outputs the superimposed signal from an output terminal b to the processor 26. This enables an observation image on which an expanded or contracted condition of each balloon 60, 80 is superimposed to be displayed on the monitor 50 of Fig. 1.

The sequencer or control circuit 170 is also connected to a cooling fan 190 and a foot switch device 192. The cooling fan 190 is driven by turning on the power switch SW1 (see Fig. 1), and sends air into the device body 102 to prevent overheat. The foot switch device 192 includes a pedal (not shown), and is operated by an operator pressing down the pedal. The foot switch device 192 is operated to perform a pressurizing process, a depressurizing process, or a process to stop the process (a pausing process) for the first balloon 60 and the second balloon 80.

The control system for endoscope A generally includes a pressurizing pump PA1, a depressurizing pump PA2, a solenoid valve VA1 which turns on/off the air supply from the pump PA1, a solenoid valve VA2 which turns on/off the air suction by the pump PA2, a solenoid valve VA3 which changes between pressurization/depressurization, a pressure sensor SA which detects a pressure in an air tube, and an air storing section (corresponds to a buffering section) 171 which buffers changes in air pressure. The pressurizing pump PA1 and the depressurizing pump PA1 are controlled to be started/stopped by the sequencer or control circuit 170. The three solenoid valves VA1, VA2, and VA3 are controlled to be switched between them by a driving signal from the sequencer or control circuit 170.

A tube between the solenoid valve VA3 and the backflow prevention unit 112 is provided with a manifold MA arranged therein, to which a pressure sensor SA is connected. The pressure sensor SA is adapted to detect a predetermined increased pressure P₁ (e.g. a pressure higher than atmospheric pressure by 5.6 kPa), an abnormal pressure value P₂ higher than the increased pressure P₁ (e.g. a pressure higher than atmospheric pressure by 8.2 kPa), and a predetermined decreased pressure P₃ (e.g. a pressure lower than atmospheric pressure by 6.0 kPa). The pressures detected by the pressure sensor SA will be sent to the sequencer or control circuit 170 to be displayed on the pressure indicator 106.

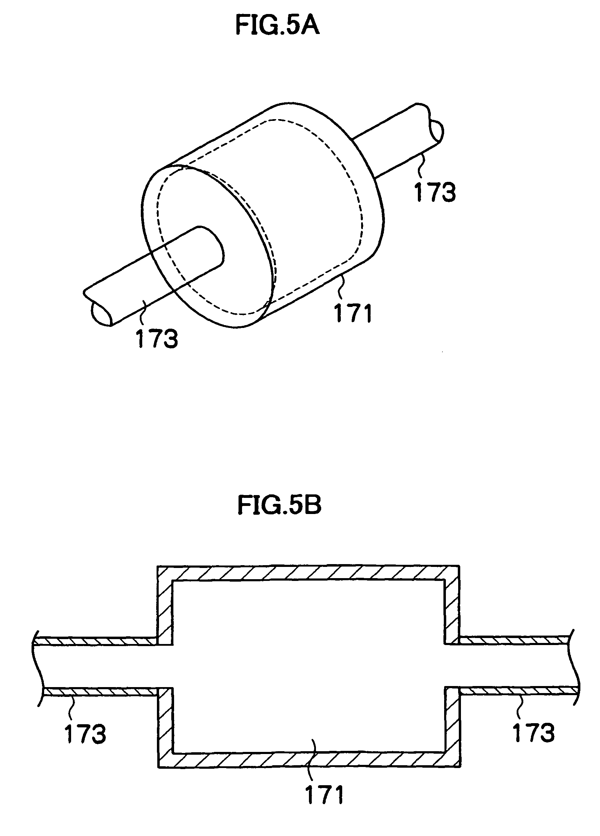

A tube path 173 between the solenoid valve VA3 and the manifold MA is provided with an air storing section 171. The air storing section 171 may be in any shape having a cross section larger than that of the tube path 173, and as shown in a perspective view of Fig. 5A and in a longitudinal sectional view of Fig. 5B for example, is formed in a cylinder having a diameter larger than that of the tube path 173. The air storing section 171 may have any size (inner volume) without limitation, but a larger size increases the buffering effect and also the device size, so the size is conveniently determined depending on a device structure. For example, for the tube path 173 having a diameter of 5 to 6 mm, the air storing section 171 is formed to have a diameter of 20 mm and a length of 50 mm. Such an air storing section 171 enables a buffering of pressure changes in the air which flows through the tube path 173.

Meanwhile, the control system for insertion assisting tool B is configured similarly to the control system for endoscope A, and generally includes a pressurizing pump PB1, a depressurizing pump PB2, a solenoid valve VB1 which turns on/off the air supply from the pump PB1, a solenoid valve VB2 which turns on/off the air suction by the pump PB2, a solenoid valve VB3 which changes between pressurization/depressurization, a pressure sensor SB which detects a pressure in the tube 120, and an air storing section (corresponds to a buffering section) 172 which buffers changes in air pressure. The pressurizing pump PB 1 and the depressurizing pump PB2 are controlled to be started/stopped by the sequencer or control circuit 170. The three solenoid valves VB1, VB2, and VB3 are controlled to be switched between them by a driving signal from the sequencer or control circuit 170.

A tube path between the solenoid valve VB3 and the backflow prevention unit 112 is provided with a manifold MB, to which a pressure sensor SB is connected. The pressure sensor SB is adapted to detect a predetermined increased pressure P₁ (e.g. a pressure higher than atmospheric pressure by 5.6 kPa), an abnormal pressure value P₂ higher than the increased pressure P₁ (e.g. a pressure higher than atmospheric pressure by 8.2 kPa), and a predetermined decreased pressure P₃ (e.g. a pressure lower than atmospheric pressure by 6.0 kPa). The pressures detected by the pressure sensor SB will be sent to the sequencer or control circuit 170 to be displayed on the pressure indicator 108.

A tube path 174 between the solenoid valve VB3 and the manifold MB is provided with an air storing section 172. The air storing section 172 may be in any shape having a cross section larger than that of the tube path 174, and for example a section similar to the air storing section 171 shown in Figs. 5A and B is used. Such an air storing section 172 enables a buffering of pressure changes in the air which flows through the tube path 174.

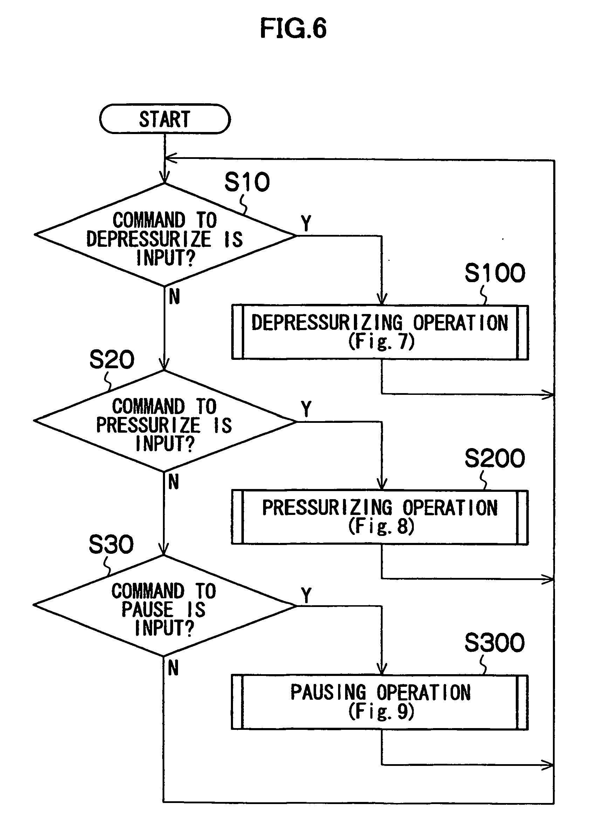

Now, operations of the sequencer or control circuit 170 will be explained with reference to the flow charts of Fig. 6 to Fig. 9. The way in which a balloon on the endoscope is controlled by the sequencer or control circuit 170 and the way in which a balloon on the insertion assisting tool is controlled by the sequencer or control circuit 170 are the same, so only the control of the balloon on the endoscope will be explained below.

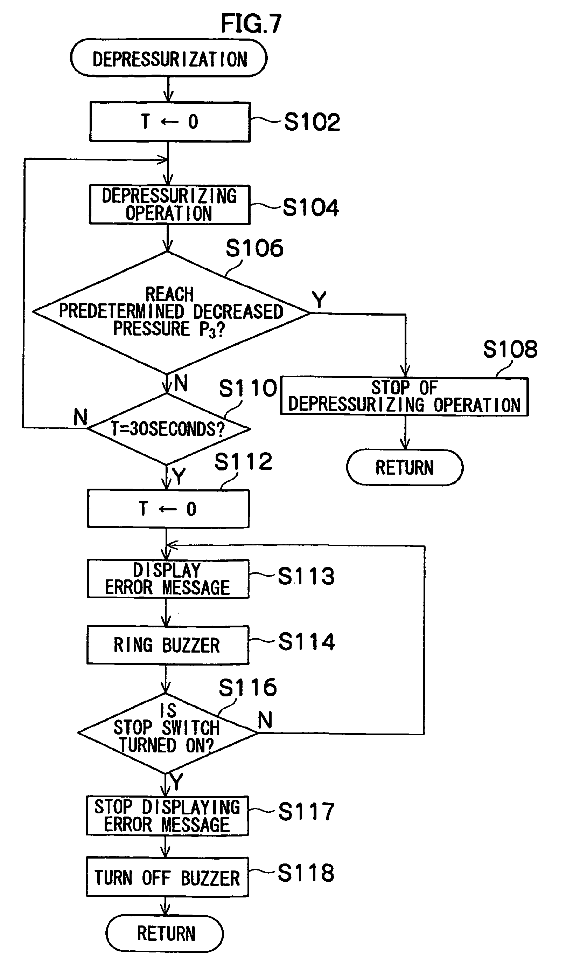

Fig. 6 is a flow chart to schematically show the operations of the sequencer or a control circuit 170. The sequencer or control circuit 170 determines if a command to depressurize the first balloon 60 (i.e. to turn off the switch SW6) is input from the hand held switch 104 or not (step S10). When the command to depressurize is input, a depressurizing operation shown in Fig. 7 is performed.

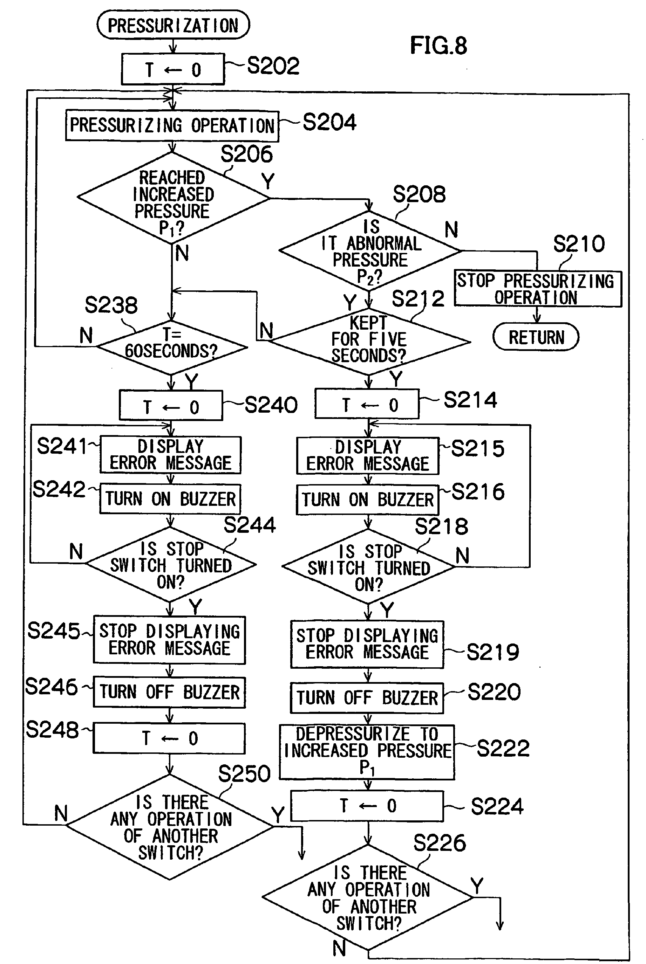

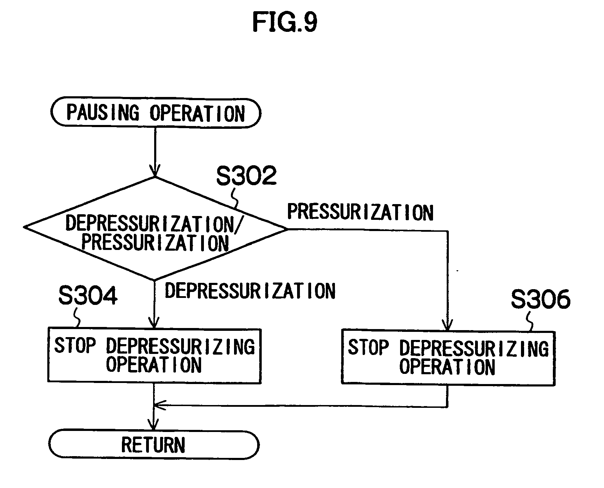

Similarly, the sequencer or control circuit 170 determines if a command to pressurize the first balloon 60 (i.e. to turn on the switch SW6) is input from the hand held switch 104 or not, and if a pausing command to hold the pressure in the balloon 60 (i.e. to turn on the pausing switch SW7) is input or not (steps S20 and S30). When the command to pressurize is input, a pressurizing operation shown in Fig. 8 is performed, and when the pausing command is input, a pausing operation shown in Fig. 9 is performed.

On the key tops of the switch SW6 and the pausing switch SW7 are provided with a green LED and a white LED respectively which are lighted when the switches are turned on. The switch SW8 and the pausing switch SW9 are also provided with a green LED and a white LED respectively.

Now, a depressurizing process will be explained with reference to the flow chart of Fig. 7.

First, the sequencer or control circuit 170 resets the time T of a timer, which measures a period of time, to 0 (step S 102), and causes the control system A to be operated to perform a depressurizing operation (step S104). That is, the solenoid valve VA1 is turned off, the solenoid valve VA2 is turned on, the solenoid valve VA3 is changed to a depressurizing mode, and the depressurizing pump PA2 is driven, all of these elements being shown in Fig. 4.

Then, the sequencer or control circuit 170 determines if the pressure in the tube 110 reaches a predetermined decreased pressure P₃ or not based on a detected signal by the pressure sensor SA (step S106), and when the pressure reached the decreased pressure P₃, the sequencer or control circuit 170 stops the depressurizing operation (step S 108). The depressurizing operation is stopped by turning off the solenoid valve VA2.

In this embodiment, because the backflow prevention unit 112 which causes a large pressure loss is disposed in a tube path, when an air suction (depressurization) is started, the pressure in the tube path 173 reaches the decreased pressure P₃ before the pressure in the first balloon 60 reaches the same, and the depressurizing operation is stopped. However, if the pressure in the first balloon 60 has not reached the decreased pressure P₃ yet, the pressure in the tube path 173 will be increased again above the decreased pressure P₃. In this case, the sequencer or control circuit 170 resumes the depressurizing operation based on the detected signal from the pressure sensor SA. In this way, the depressurizing operation is stopped and resumed a plurality of times, so that the pressure in the first balloon 60 can reach the predetermined decreased pressure P₃. In this embodiment, as will be explained below, because the air storing section 171 enables the difference between the pressure in the tube path 173 and the pressure in the first balloon 60 to be reduced, the number of times to repeat the depressurizing operation can be reduced, which makes the pressure in the first balloon 60 rapidly reach a preset decreased pressure P₃.

On the contrary, if the first balloon 60 does not reach a decreased pressure P₃, the sequencer or control circuit 170 determines if the time T has passed more than thirty seconds or not since the depressurizing operation started (step S110). In the case where the steps S 104, S106, and S110 are repeated until the time T passes more than thirty seconds, the sequencer or control circuit 170 determines that some disorder exists (for example, the tube 110 is not connected to the air inlet for balloon 38).

When a disorder is detected as described above, the time T of the timer is reset to 0, an error message is displayed, and a buzzer BZ is rung at the same time (S 112, S 113, and S114). The error message is displayed as an error code (e.g. "Err7") alternately with a pressure value of the first balloon 60 at a pressure indicator 106. Simultaneously, the red LEDs which are disposed on each key top of the stop switch SW2 at the device body 102 and the stop switch SW3 at the hand held switch 104 are lighted.

After that, the sequencer or control circuit 170 determines if either of the stop switches SW2 or SW5 is pressed or not (step S116), and if pressed, the displayed error message is cleared, and the ringing buzzer BZ is stopped (steps S117 and S118). If neither of the stop switch SW2 or SW5 is pressed, the error message is displayed and the buzzer BZ is kept ringing.

When a disorder is reported by the buzzer BZ for example during the above depressurizing operation, usually, an operator of the double balloon endoscope presses the stop switch SW2 or SW5, and then check if the tube 110 is normally connected, for example.

Now, a pressurizing process will be explained with reference to the flow chart of Fig. 8.

First, the sequencer or control circuit 170 resets the time T of a timer, which measures a period of time, to 0 (step S202), and causes the control system A to be operated to perform a pressurizing operation (step S204). That is, the solenoid valve VA1 is turned on, the solenoid valve VA2 is turned off, the solenoid valve VA3 is changed to a pressurizing mode, and the pressurizing pump PA1 is driven.

Then, the sequencer or control circuit 170 determines if the pressure in the tube 110 reaches a preset increased pressure P₁ or not based on a detected signal by the pressure sensor SA (step S206), and when the pressure reached the increased pressure P₁, the sequencer or control circuit 170 determines if the pressure reaches an abnormal pressure P₂ or not (step S208). If the pressure does not reach the abnormal pressure P₂, the pressurizing operation is stopped (step S210). The pressurizing operation is stopped by turning off the solenoid valve VA1.

In this embodiment, because the backflow prevention unit 112 which causes a large pressure loss is disposed in the tube path, when an air supply (pressurization) is started, the pressure in the tube path 173 reaches the increased pressure P₁ before the pressure in the first balloon 60 reaches the same, and the pressurizing operation is stopped. However, if the pressure in the first balloon 60 has not reached the increased pressure P₁ yet, the pressure in the tube path 173 will be decreased again below the increased pressure P₁. In this case, the sequencer or control circuit 170 resumes the pressurizing operation based on the detected signal by the pressure sensor SA. In this way, the pressurizing operation is stopped and resumed a plurality of times, so that the pressure in the first balloon 60 can reach the predetermined increased pressure P₁. In this embodiment, as will be explained below, because the air storing section 171 enables the difference between the pressure in the tube path 173 and the pressure in the first balloon 60 to be reduced, the number of times to repeat the pressurizing operation can be reduced, which makes the pressure in the first balloon 60 rapidly reach a predetermined increased pressure P₁.

On the contrary, if the peristaltic movement of small intestine is evoked or the pressurizing operation is not stopped due to some disorder of the device body 102 (e.g. a disorder of the solenoid valve VA1), the pressure in the tube 110 may reach an abnormal pressure P₃. In this case, the process goes from step S208 to step S212, and at step S212, the sequencer or control circuit 170 determines if the abnormal pressure P₃ has been kept for five seconds or not.

When the abnormal pressure P₃ has been kept for five seconds, the time T of the timer is reset to 0, an error message is displayed, and a buzzer BZ is rung at the same time (steps S214, S215, and S216). The error message is displayed as an error code (e.g. "Err4") alternately with a balloon pressure value at the pressure indicator 106.

After that, the sequencer or control circuit 170 determines if either of the stop switch SW2 or SW5 is pressed or not (step S218), and if pressed, the displayed error message is cleared and the ringing buzzer BZ is stopped (steps S219 and S220). Then the depressurizing operation is repeated until the abnormal pressure P₂ reaches the increased pressure P₁ (step S222). The depressurizing operation is performed by switching the solenoid valve VA3 to a depressurizing mode. In this case, for example if a pressurizing operation cannot be stopped due to a disorder of the solenoid valve VA1, a switching of the solenoid valve VA3 enables the depressurization.

Then the time T of the timer is reset to 0 (step S224), and it is determined if there is any operation of another switch SW or not such as a turning off (depressurization) of the switch SW6 (step S226). At step S226, when it is determined that there is an operation of another switch SW, a balloon control is performed based on the command by the switch SW.

At step S218, if neither of the stop switch SW2 or SW5 is pressed, the error message is displayed and the buzzer BZ is kept ringing.

Back to step S206, if the pressure in the tube 110 does not reach the increased pressure P₁ during the pressurizing operation, the sequencer or control circuit 170 determines if the time T has passed more than sixty seconds or not since the pressurizing operation started (step S238). In the case where the steps S204, S206, and S238 are repeated until the time T passes more than sixty seconds, the sequencer or control circuit 170 determines that some disorder exists (for example, the tube 110 is not connected to the air inlet for balloon 38).

When a disorder is detected as described above, the time T of the timer is reset to 0, an error message is displayed, and a buzzer BZ is rung at the same time (step S240 and S242). The error message is displayed as an error code (e.g. "Err5") alternately with a balloon pressure value at a pressure indicator 106.

After that, the sequencer or control circuit 170 determines if either of the stop switch SW2 or SW5 is pressed or not (step S244), and if pressed, the displayed error message is cleared, and the ringing buzzer BZ is stopped (steps S246). Then the time T of the timer is reset to 0 (step S248), and it is determined if there is any operation of another switch SW or not (step S250). When it is determined that there has been no operation of another switch SW for twenty seconds since the buzzer BZ stopped, the pressurizing operation is continued. At step S250, when it is determined that there is an operation of another switch SW, a balloon control is performed based on the command by the switch SW.

At step S244, if neither of the stop switch SW2 or SW5 is pressed, the error message is displayed and the buzzer BZ is kept ringing.

Now, a method for detecting a disorder of the burst of the first balloon 60 will be explained.

The air supply tube which is disposed along the inserting section 12 of the balloon endoscope 10 has a diameter (about 0.8 mm) which is smaller compared to the diameter of the tube 110 (about 6 mm), and to the coupling between the tube 110 and the device body 102 is disposed the backflow prevention unit 112 which is formed of a filter causing a large pressure loss. Due to this configuration, upon an air supply (pressurization), the pressure of the tube path in the device body 102 reaches an increased pressure P₁ before the pressure in the first balloon 60 reaches the same, and the pressurizing operation is stopped. However, if the pressure in the first balloon 60 has not reached the increased pressure P₁, the air in the tube path in the device body 102 will be supplied to the first balloon 60 via the air supply tube, and the pressure of the tube path in the device body 102 is decreased again below the increased pressure P₁. In this case, the sequencer or control circuit 170 resumes the pressurizing operation based on the detected signal by the pressure sensor SA.

With the first balloon 60 being not burst, the above pressurizing operation is stopped and resumed a plurality of times, so that the pressure in the first balloon 60 can reach the increased pressure P₁; however, with the first balloon 60 being burst, the pressure in the first balloon 60 cannot reach the increased pressure P₁ even after a repetition of the pressurizing operation for a long time.

So, the sequencer or control circuit 170 determines that the first balloon 60 is burst when a repetition of start and stop of the pressurizing operation in a short cycle (i.e. a chattering of turning on/off of the solenoid valve VA1) is continued for a period of time which is much longer than a chattering period for the pressurizing operation on the normal contracted first balloon 60 (e.g. for 40 seconds), so that the error message is displayed and the buzzer BZ is rung. The error message is displayed as an error code (e.g. "Err5") alternately with a balloon pressure value at a pressure indicator 106.

Now, a pausing process will be explained with reference to the flow chart of Fig. 9.

The sequencer or control circuit 170 determines during which of the depressurizing operation or the pressurizing operation a pausing command to hold the pressure in the first balloon 60 (a turning on of the pausing switch SW7) is input (step S302). If a pausing command is input during the depressurizing operation, the solenoid valve VA2 is switched to stop the depressurizing operation (step S304).

If a pausing command is input during the pressurizing operation, the solenoid valve VA1 is switched to stop the pressurizing operation (step S306).

This pausing function is used, for example, when a double balloon endoscope is inserted into large intestine with a balloon being expanded. That is, in the lumen of large intestine having a diameter larger than that of the lumen of small intestine, the pressure in a balloon may not reach a predetermined increased pressure P₁ even when the balloon is expanded to the size of the lumen, and in this case, the above pausing function is used to stop the pressurizing operation.

Pressing down the pausing switch SW7 again during the depressurizing or pressurizing operation, which has been temporarily paused, causes the original depressurizing or pressurizing operation to be resumed. Also, if the depressurizing or pressurizing switch (an endoscope turning on/off switch SW6) is pressed down during the depressurizing or pressurizing operation, which has been temporarily paused, the operation specified by the pressed switch will have a priority.

Now, operations of the balloon control device 100 of this embodiment will be explained.

As described above, because the backflow prevention units 112 and 122 include filters for gas and liquid separation so that air passes through the filters, the backflow prevention units 112 and 122 cause a large pressure loss. Thus, in conventional apparatuses (that is, without air storing sections 171 and 172), upon the driving of pumps PA1, PA2, PB1 and PB2, a pressure in a tube path upstream of the backflow prevention units 112 and 122 toward the pumps rapidly changes, while a pressure in a tube path downstream of the backflow prevention units 112 and 122 toward the balloons slowly changes. So, the conventional apparatus had a problem that the above configuration causes a difference between the measured values obtained by the pressure sensors SA and SB and the real pressures in the balloons 60, 80, and that the inner pressures of the balloons 60, 80 cannot be accurately controlled. Especially, in a pressurizing operation, because the pressure in the tube path upstream of the backflow prevention units 112 and 122 toward the pumps is suddenly increased, the measured values obtained by the pressure sensors SA and SB reach an increased pressure P₁ before the real inner pressures in the balloons 60, 80 reach the same. Thus the conventional apparatus had another problem that every time this happens, the pumps PA 1 and PB 1 are stopped, and that it takes a long time to make the real inner pressures of the balloons 60, 80 reach the increased pressure P₁.

To the contrary, the embodiment of the present invention comprises the air storing sections 171, 172 in the tube paths 173, 174 between the pumps PA1, PA2, PB1 and PB2 and the manifolds MA and MB, so that, upon the driving of pumps PA1, PA2, PB 1 and PB2, the pressure changes in the air flowing through the tube paths 173, 174 are buffered by the air storing sections 171, 172 which have large inner volumes. In this way, as the rapid change in pressure in the tube paths upstream of the air storing sections 171, 172 toward the balloons 60, 80 is restrained, the differences between the pressures in the tube paths and the balloons 60, 80 can be reduced. This in turn reduces errors between the measured values obtained by the pressure sensors SA and SB and the real inner pressures of the balloons 60, 80, thereby the inner pressures of the balloons 60, 80 can be accurately controlled by controlling the expansion and contraction of the balloons 60, 80 based on the measured values obtained by the pressure sensors SA and SB.

According to the balloon control device 100 of this embodiment, as the air storing sections 171, 172 are provided in the tube paths 173, 174 of the device body 102, errors between the measured values obtained by the pressure sensors SA and SB and the real inner pressures of the balloons 60, 80 can be reduced, and the inner pressures of the balloons 60, 80 can be accurately controlled. This reduces the number of times to repeat the stopping and resuming of the pressurizing or depressurizing operations in the pressurizing or depressurizing process described above, with the result that the inner pressures of balloons 60, 80 can rapidly reach a preset increased pressure P₁ or decreased pressure P₃.

In the above embodiment, the air storing sections 171, 172 are formed into cylindrical shape, but the shape of the air storing sections 171, 172 is not limited to this, and the air storing sections 171, 172 may be in any shape which buffers a change in pressure of a fluid through the tube path 173, 174. Thus, the air storing sections 171, 172 may be formed into hollow boxes or hollow spheres. The air storing sections 171, 172 also may be formed of rubber bags which are freely expandable and contractible.

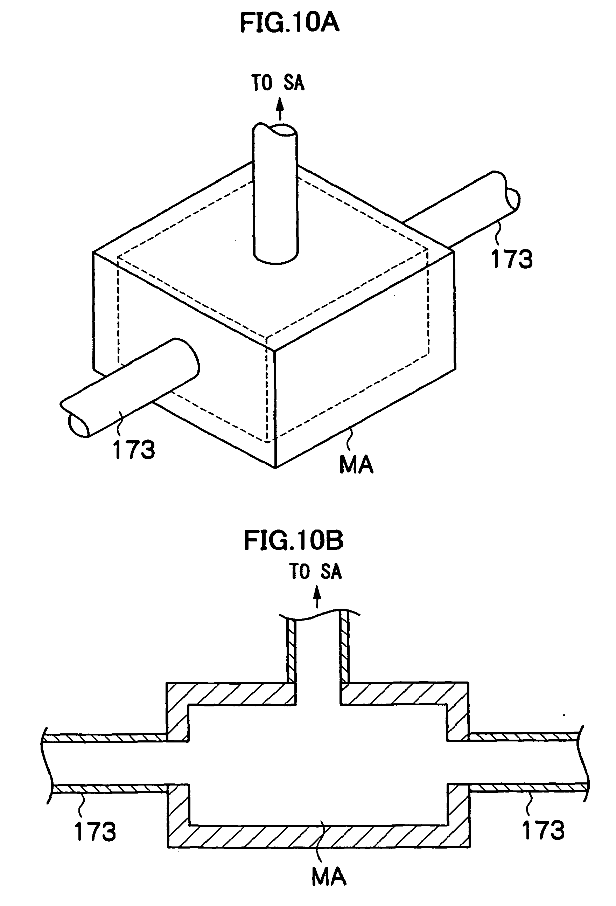

In addition, the air storing sections 171, 172 may be formed using other elements. For example as shown in the perspective view of Fig. 10A and in the longitudinal sectional view of Fig. 10B, the air storing sections 171, 172 may be provided by a manifold MA (or MB) which has a hollow inner space.

In the above embodiment, the air storing sections 171, 172 are disposed in the tube paths 173, 174 which connect the manifolds MA, MB to the solenoid valves VA3, VB3, but the arrangement of the air storing sections 171, 172 is not limited to this, and the air storing sections 171, 172 may be arranged anywhere in a tube path between a measured location by the pressure sensors SA and SB (manifolds MA and MB) and each pump PA1, PA2, PB1 and PB2.

The above embodiment has been explained as an example of the balloon control device 100 which is applied to a double balloon endoscopic apparatus having a first balloon 60 and a second balloon 80, but the present invention is not limited to this, and the present invention may be applied to a balloon control device of an endoscopic apparatus having only one balloon.

## Claims

1. A balloon control device for an endscopic apparatus (10), which supplies and sucks a fluid to and from either a balloon (60) mounted to an inserting section (12) of the endscopic apparatus (10) or a balloon (80) mounted to an insertion assisting tool (70) covering the inserting section (12) for assisting the insertion of the inserting section (12), comprising
a buffering section (171, 172) in a tube path (173, 174) through which a fluid flows so as to buffer a change in pressure of the fluid,
**characterised in that**
a pressure sensor (SA, SB) is connected to the tube path (173, 174) through which the fluid flows via a manifold (MA, MB), and the buffering section (171, 172) is provided by the manifold (MA, MB).

2. The balloon control device for an endscopic apparatus (10) according to claim 1, wherein
a filter for gas and liquid separation is disposed in the tube path (173, 174), and the buffering section (171, 172) is disposed across the filter on a side opposite to the balloon (60, 80).

3. The balloon control device for an endscopic apparatus (10) according to any one of claims 1 to 2, wherein
the buffering section (171, 172) is provided within the device body.

## Patentansprüche

1. Ballon-Steuervorrichtung für ein endoskopisches Gerät (10), welche ein Fluid zu oder von einem Ballon (60), welcher an einem Einführabschnitt (12) des endoskopischen Gerätes (10) angebracht ist, oder einem Ballon (80), welcher an einem Einführungs-Unterstützungs-Werkzeug (70) angebracht ist, welches den Einführabschnitt (12) zum Unterstützen des Einführens des Einführabschnittes (12) überdeckt, zuführt und entnimmt, umfassend
einen Pufferabschnitt (171, 172) in einer Rohrstrecke (173, 174), durch welche ein Fluid strömt, um eine Druckänderung des Fluids zu puffern,
**dadurch gekennzeichnet, dass**
ein Drucksensor (SA, SB) mit der Rohrstrecke (1 73, 1 74), durch welche das Fluid strömt, über eine Verzweigung (MA, MB) verbunden ist und der Pufferabschnitt (171, 172) durch die Verzweigung (MA, MB) geschaffen wird.

2. Ballon-Steuervorrichtung für ein endoskopisches Gerät (10) nach Anspruch 1, wobei
ein Filter für Gas- und Flüssigkeits-Trennung in der Rohrstrecke (173, 1 74) angeordnet ist und der Pufferabschnitt (1 71 , 172) jenseits des Filters auf einer dem Ballon (60, 80) gegenüberliegenden Seite angeordnet ist.

3. Ballon-Steuervorrichtung für ein endoskopisches Gerät (10) nach einem der Ansprüche 1 bis 2, wobei
der Pufferabschnitt (171, 172) innerhalb des Vorrichtungsgehäuses vorgesehen ist.

## Revendications

1. Dispositif de commande de ballon pour un appareil endoscopique (10), lequel permet l'alimentation d'un fluide et l'aspiration de celui-ci à destination et en provenance soit d'un ballon (60) monté sur une partie d'insertion (12) de l'appareil endoscopique (10), soit d'un ballon (80) monté sur un outil permettant l'insertion (70) et couvrant la partie d'insertion (12) en vue de permettre l'insertion de la partie d'insertion (12), comprenant:
une partie formant tampon (171, 172) dans un trajet de tube (173, 174) dans lequel un fluide s'écoule, de manière à réguler une modification de la pression du fluide,
**caractérisé en ce que**
un détecteur de pression (SA, SB) est relié au trajet de tube (173, 174) dans lequel s'écoule le fluide par un branchement (MA, MB), et la partie formant tampon (171, 172) est fournie par le branchement (MA, MB).

2. Dispositif de commande de ballon pour un appareil endoscopique (10) selon la revendication 1, dans lequel
un filtre de séparation des gaz et liquides est disposé dans le trajet de tube (173, 174), et la partie formant tampon (171, 172) est disposée au-delà le filtre sur un coté opposé au ballon (60, 80).

3. Dispositif de commande de ballon pour un appareil endoscopique (10) selon l'une quelconque des revendications 1 ou 2, dans lequel
la partie formant tampon (171, 172) est prévue à l'intérieur du corps de dispositif.
